# EUROPEAN PATENT APPLICATION

(11) **EP 3 951 387 A1**
(43) Date of publication of application: **09.02.2022**
(21) Application number: 19923273.7
(22) Date of filing: 29.03.2019
(51) Int. Cl.: G01N 33/53, C07K 16/18, C07K 16/24, G01N 33/68

(54) **ARTERIOSCLEROSIS AND ARTERIOSCLEROSIS-RELATED DISEASE MARKER**

(71) Applicant: EIKEN KAGAKU KABUSHIKI KAISHA, Tokyo 110-8408 (JP); National Cerebral and Cardiovascular Center, Suita-shi, Osaka 564-8565 (JP)
(72) Inventor: MINAMINO, Naoto, Suita-shi, Osaka 564-8565 (JP); YAGI, Hiroaki, Suita-shi, Osaka 564-8565 (JP); NISHIGORI, Mitsuhiro, Suita-shi, Osaka 564-8565 (JP); UEDA, Hatsue, Suita-shi, Osaka 564-8565 (JP); MATSUDA, Hitoshi, Suita-shi, Osaka 564-8565 (JP); MURAKAMI, Yusuke, Shimotsuga-gun , Tochigi 329-0114 (JP)
(74) Representative: Vossius & Partner Patentanwälte Rechtsanwälte mbB
(86) International application number: PCT/JP2019/013958
(87) International publication number: WO 2020/202241

(57) **Abstract**

The present invention is intended to provide a novel biomarker for detecting arteriosclerosis-related disease or for evaluating the stage of progression of arteriosclerosis, and specifically, the present invention relates to a marker for detecting arteriosclerosis-related disease or for evaluating the stage of progression of arteriosclerosis, comprising an NPC2 (Niemann-Pick disease type C2) protein and/or an IGFBP7 (Insulin-like growth factor-binding protein 7) protein.

## Description

### Technical Field

The present invention relates to the technical field of a clinical test agent that assists the diagnosis of arteriosclerosis-related disease and arteriosclerosis. More specifically, the present invention relates to a biomarker of arteriosclerosis-related disease, a biomarker that reflects the condition of arteriosclerosis (hereinafter also referred to as "stage of progression"), and a measurement reagent, a reagent kit, a method for detecting arteriosclerosis-related disease, and a method for evaluating the stage of progression of arteriosclerosis, in all of which the aforementioned markers are used.

### Background Art

Arteriosclerosis is a generic term of a state, in which various substances are deposited inside the artery, and the inner wall thereof becomes thick or hardened, so that the wall loses elasticity or the lumen is narrowed. Arteriosclerosis is classified into three types of arteriosclerosis, namely, atheromatous arteriosclerosis (hereinafter referred to as "atherosclerosis"), medial calcific sclerosis, and arteriolosclerosis. In general, however, the term "arteriosclerosis" means atherosclerosis in many cases. Atherosclerosis is arteriosclerosis occurring in relatively thick artery such as aorta, cerebral artery and coronary artery, and it is said that atheromatous substances consisting of fats such as cholesterols are accumulated in the intima of artery to form atheroma (atherosclerotic plaque), and that the gradual thickening of the atheroma narrows the lumen of artery. Moreover, the thus formed, unstable atheroma is peeled to form blood clots, which cause various types of infarction diseases in many cases. Thus, it has been known that arteriosclerosis causes diseases such as myocardial infarction, peripheral arterial disease, aortic aneurysm, aortic dissection, stroke (atherothrombotic cerebral infarction, transient ischemic attack, etc.), renal artery stenosis, internal carotid artery stenosis, and angina pectoris. With aging of population and changes in lifestyle, arteriosclerosis has been increased in recent years. Arteriosclerosis is a serious disease, which progresses without any symptoms, and which then leads to a life-threatening situation, if myocardial infarction, cerebral infarction, aortic aneurysm or the like is developed. However, since the onset of such diseases can be avoided by performing a suitable treatment on arteriosclerosis, it is extremely important to detect arteriosclerosis or arteriosclerotic disease at an early stage or to control the stage of progression of arteriosclerosis.

In almost all cases, thoracic aortic aneurysm that is developed due to, for example, arteriosclerosis, is found, by chance, when a chest X-ray image is taken in a medical checkup and the like. In the case of abdominal aortic aneurysm, it is often found as a pulsing lump when the abdomen is palpated to diagnose digestive disease such as gastric ulcer or cholelithiasis, or during the abdominal echo (ultrasonic wave) examination of digestive tract, kidney, prostate, etc., or during the MRI examination of the chest and abdomen.

As markers of arteriosclerosis or arteriosclerosis-related disease, LDL cholesterol used in risk assessment, lipid markers such as oxidized LDL, and inflammatory markers such as CRP and Pentraxin3 have been known (Non Patent Literature 1). However, it cannot be said that all of these markers are sufficient in terms of sensitivity, specificity, and disease specificity.

On the other hand, as means for noninvasively grasping the condition of arteriosclerosis, the measurement of blood vessel intima-media thickness (hereinafter referred to as "IMT") using carotid ultrasonography, the measurement of a brachial-ankle pulse wave velocity (hereinafter referred to as "baPWV") and the like have been known (Non Patent Literature 2). Since these examinations are capable of noninvasively simply examining the stage of progression of arteriosclerosis for a necessary period of time such as more than a dozen minutes in many cases, these examinations are carried out in many hospitals. However, these examinations are apparently unsuitable compared with blood tests and the like, in that these examinations can be carried out only in well-equipped hospitals, and also in that these examinations have poor throughput, when used in a screening test for selecting a high arteriosclerosis risk group from a large population.

### Citation List

### Non Patent Literature

Non Patent Literature 1: S. Hirayama, etal., Vascular Medicine, 10(1), 10-15, 2014
Non Patent Literature 2: Guidelines regarding Diagnosis and Treatment of Cardiovascular Diseases (Joint working group report, 2011-2012), Guidelines for Non-Invasive Vascular Function Test, 2013

### Summary of Invention

### Technical Problem

Blood pressure and blood tests such as LDL cholesterol and HDL cholesterol, which have been used as risk factors of arteriosclerosis in screening tests performed in medical checks and the like so far, only show numerical values at the time point of these tests, and thus, these factors do not reflect the conditions of the tissues or cells of blood walls that are exposed to various risk factors or stresses at a different strength and/or for a different period of time. Taking into consideration the aforementioned current circumstances, it is anticipated that the development of a biomarker, which shows a strong correlation with methods for evaluating arteriosclerosis, such as carotid ultrasonography or baPWV examination, and can be utilized in screening, will bring a new phase for prevention of arteriosclerosis, the diagnosis of the onset of arteriosclerosis-related disease, evaluation of the stage of progression thereof, the development of a therapeutic agent, etc.

It is an object of the present invention to provide a novel biomarker that reflects the condition of arteriosclerosis and is capable of detecting arteriosclerosis-related disease or evaluating the stage of progression of the arteriosclerosis, simply, with high sensitivity.

### Solution to Problem

In order to achieve the purpose for providing an excellent arteriosclerotic disease marker that reflects the condition of arteriosclerosis, the present inventors have performed proteome analysis, using the tissues of atherosclerotic thoracic aortic aneurysm as a disease caused by atherosclerosis. Since atherosclerotic thoracic aortic aneurysm is developed due to atherosclerosis and further, the affected site of atherosclerotic thoracic aortic aneurysm can be clearly distinguished from other sites, this disease is extremely suitable for the analysis of arteriosclerosis-related disease using tissues. From the obtained protein profile, the tissues can be classified depending on the stage of disease progression, and changes in the known structural proteins or changes in proteases and the like can be well explained by using those classifications. Furthermore, the proteome analysis was also performed on the aortic tissues of (non-vascular disease) control subjects in the same manner as above. In order to identify factors associated with the onset of the disease using the obtained data, a comparison was made between the proteome data of the aortic tissues of control subjects and the proteome data of the tissues of atherosclerotic thoracic aortic aneurysm patients. Further, in order to identify factors associated with the disease progression, a comparison was made among individual proteome analysis data of the tissues of different classifications of atherosclerotic thoracic aortic aneurysm patients. It is assumed that factors that fluctuate in both of the comparisons would be associated with the onset and/or disease progression. Among the factors fluctuating in both of the comparisons, factors that fluctuate even in blood were searched, and then, a novel marker that reflects, with high precision, the onset and progression of atherosclerosis, that are, the onset of arteriosclerosis-related disease and the stage of progression of atherosclerosis has been found, thereby completing the present invention.

Specifically, the present invention consists of the following components.
(1) A method for detecting arteriosclerosis-related disease, comprising a step of measuring the following 1) and/or 2) in a sample obtained from a subject:
   1) an NPC2 (Niemann-Pick disease type C2) protein, and/or
   2) an IGFBP7 (Insulin-like growth factor-binding protein 7) protein.
(2) A method for detecting or screening the recurrence of arteriosclerosis-related disease, comprising a step of measuring the following 1) and/or 2) in a sample obtained from a subject having a risk of the recurrence of arteriosclerosis-related disease:
   1) an NPC2 (Niemann-Pick disease type C2) protein, and/or
   2) an IGFBP7 (Insulin-like growth factor-binding protein 7) protein.
(3) A method for detecting or screening a subject having a risk of developing arteriosclerosis-related disease in future, comprising a step of measuring the following 1) and/or 2) in a sample obtained from the subject:
   1) an NPC2 (Niemann-Pick disease type C2) protein, and/or
   2) an IGFBP7 (Insulin-like growth factor-binding protein 7) protein.
(4) A method for evaluating the effects of a preventive or therapeutic agent for arteriosclerosis-related disease or arteriosclerosis, wherein the method comprises: a step of measuring the following 1) and/or 2) in a sample obtained from a subject in need of the prevention or treatment of arteriosclerosis-related disease or arteriosclerosis, before administration of a preventive or therapeutic agent for arteriosclerosis-related disease or arteriosclerosis; and a step of measuring the following 1) and/or 2) in a sample obtained from the subject, after administration of the preventive or therapeutic agent for arteriosclerosis-related disease or arteriosclerosis, or the method comprises a step of measuring the following 1) and/or 2) in samples obtained, at two or more time points in chronological order, from a subject in need of the prevention or treatment of arteriosclerosis-related disease or arteriosclerosis, after administration of a preventive or therapeutic agent for arteriosclerosis-related disease or arteriosclerosis:
   1) an NPC2 (Niemann-Pick disease type C2) protein, and/or
   2) an IGFBP7 (Insulin-like growth factor-binding protein 7) protein.
(5) The method according to any one of the above (1) to (4), wherein the arteriosclerosis-related disease is selected from the group consisting of myocardial infarction, peripheral arterial disease, aortic aneurysm, aortic dissection, atherothrombotic cerebral infarction, transient ischemic attack, renal artery stenosis, internal carotid artery stenosis, and angina pectoris.
(6) A method for evaluating the stage of progression of arteriosclerosis comprising a step of measuring the following 1) and/or 2) in a sample obtained from a subject:
   1) an NPC2 (Niemann-Pick disease type C2) protein, and/or
   2) an IGFBP7 (Insulin-like growth factor-binding protein 7) protein.
(7) The method according to any one of the above (1) to (6), wherein the sample is selected from the group consisting of serum, plasma, blood, urine, and saliva.
(8) The method according to any one of the above (1) to (7), wherein the subject is an animal in need of the prevention or treatment of arteriosclerosis-related disease or arteriosclerosis, and the effects of the preventive or therapeutic agent for arteriosclerosis-related disease or arteriosclerosis are evaluated or determined using the NPC2 protein and/or the IGFBP7 protein as an indicator.
(9) The method according to any one of the above (1) to (8), wherein the subject is a human.
(10) The method according to any one of the above (1) to (9), wherein the measurement method is immunoassay or mass spectrometry.
(11) A marker for detecting arteriosclerosis-related disease, comprising the following 1) and/or 2):
   1) an NPC2 protein, and/or
   2) an IGFBP7 protein.
(12) The marker according to the above (11), wherein the arteriosclerosis-related disease is selected from the group consisting of myocardial infarction, peripheral arterial disease, aortic aneurysm, aortic dissection, atherothrombotic cerebral infarction, transient ischemic attack, renal artery stenosis, internal carotid artery stenosis, and angina pectoris.
(13) A marker for evaluating the stage of progression of arteriosclerosis, comprising the following 1) and/or 2):
   1) an NPC2 protein, and/or
   2) an IGFBP7 protein.
(14) A measurement reagent for detecting arteriosclerosis-related disease or for evaluating the stage of progression of arteriosclerosis, wherein the measurement reagent comprises an antibody or an aptamer for measuring the marker according to any one of the above (11) to (13).
(15) The measurement reagent according to the above (14), wherein the antibody is a monoclonal antibody and/or a polyclonal antibody.
(16) A reagent kit for detecting arteriosclerosis-related disease or for evaluating the stage of progression of arteriosclerosis, wherein the reagent kit includes the measurement reagent according to the above (14) or (15).
(17) The marker, measurement reagent, or reagent kit according to any one of the above (11) to (16), wherein the sample used for detecting arteriosclerosis-related disease or for evaluating the stage of progression of arteriosclerosis is selected from the group consisting of serum, plasma, blood, urine, and saliva.
(18) Use of the marker according to any one of the above (11) to (13) for screening a preventive or therapeutic agent for arteriosclerosis-related disease or arteriosclerosis.
(19) Use of the marker according to any one of the above (11) to (13) for detecting or screening *in vitro* the recurrence of arteriosclerosis-related disease.
(20) Use of the marker according to any one of the above (11) to (13) for detecting or screening *in vitro* a subject having a risk of developing arteriosclerosis-related disease in future.

### Advantageous Effects of Invention

NPC2 or IGFBP7 found by the present invention is used as a marker of arteriosclerosis-related disease (i.e. a marker for detecting arteriosclerosis-related disease) or an arteriosclerosis marker (i.e. a marker for evaluating the stage of progression of arteriosclerosis), so that the onset of arteriosclerosis-related disease or arteriosclerosis can be detected with high sensitivity. Moreover, as shown in the Examples later, NPC2 and IGFBP7 are considered to be markers that each reflect a different aspect of arteriosclerosis, and it becomes possible to obtain more detailed information of the stage of progression of arteriosclerosis by using the two markers in combination with each other.

Moreover, a measurement reagent and a measurement kit, in which the marker for detecting arteriosclerosis-related disease or the marker for evaluating the stage of progression of arteriosclerosis according to the present invention is used, can be used in the early diagnosis of the onset of arteriosclerosis-related disease or arteriosclerosis, and the grasping (monitoring) of the progression of arteriosclerosis, and thus, can contribute to avoid a risk of these diseases becoming severe by the early detection of the diseases.

Also, the marker for detecting arteriosclerosis-related disease or the marker for evaluating the stage of progression of arteriosclerosis according to the present invention can be used in the development of a preventive or therapeutic agent for these diseases and the evaluation thereof. Further, by using the marker for detecting arteriosclerosis-related disease or the marker for evaluating the stage of progression of arteriosclerosis according to the present invention, the recurrence of arteriosclerosis-related disease can be detected or screened, or a subject having a risk of developing arteriosclerosis-related disease in future can be detected or screened.

### Brief Description of Drawings

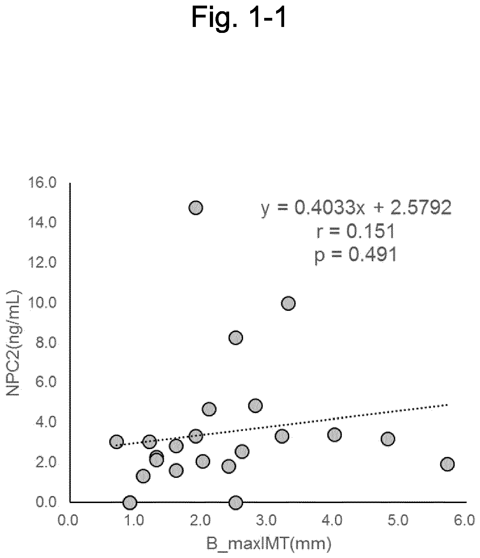

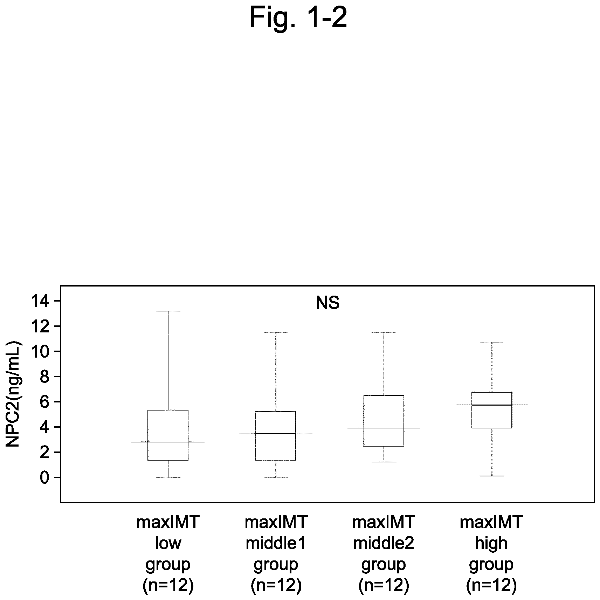

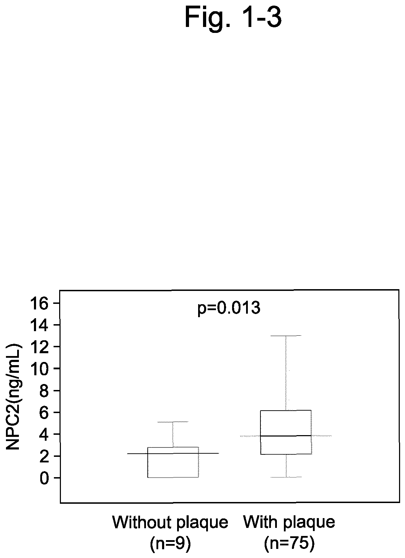

[Figure 1-1] Figure 1-1 is a graph showing the results of correlation analysis of the NPC2 value and the maxIMT value in the serum specimens of patients with arteriosclerosis-related disease (arteriosclerosis obliterans and myocardial infarction) in Example 1.
[Figure 1-2] Figure 1-2 is a graph showing the results of the trend analysis of the NPC2 value and the maxIMT value in the serum specimens of patients with aortic aneurysm (including thoracic aortic aneurysm, abdominal aortic aneurysm, and aortic aneurysm involving the complex onset in the chest and the abdomen) in Example 1. "NS" indicates "not significant (no significant difference)."
[Figure 1-3] Figure 1-3 is a graph showing the results of comparative analysis of the NPC2 value and the presence or absence of a carotid artery plaque in the serum specimens of patients with arteriosclerosis-related disease (aortic aneurysm, aortic dissection, arteriosclerosis obliterans, and myocardial infarction) in Example 1.

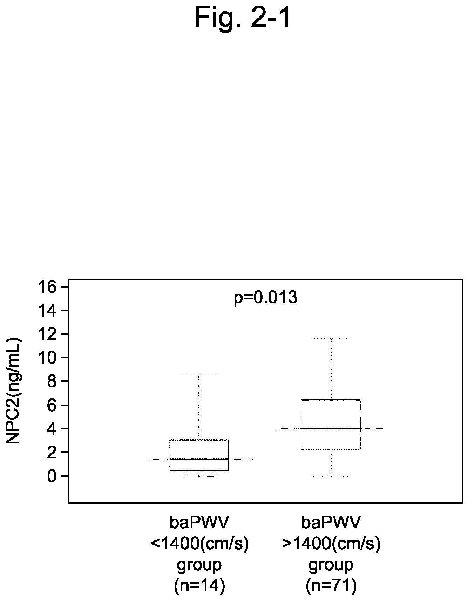

[Figure 2-1] Figure 2-1 is a graph showing the results of comparative analysis of the NPC2 value and the baPWV value (cutoff value: 1400 (cm/s)) in the serum specimens of patients with arteriosclerosis-related disease (aortic aneurysm, aortic dissection, arteriosclerosis obliterans, and myocardial infarction) in Example 2.

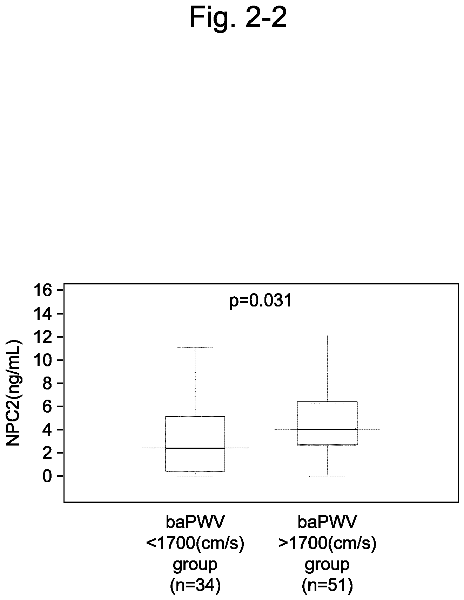

[Figure 2-2] Figure 2-2 is a graph showing the results of comparative analysis of the NPC2 value and the baPWV value (cutoff value: 1700 (cm/s)) in the serum specimens of patients with arteriosclerosis-related disease (aortic aneurysm, aortic dissection, arteriosclerosis obliterans, and myocardial infarction) in Example 2.

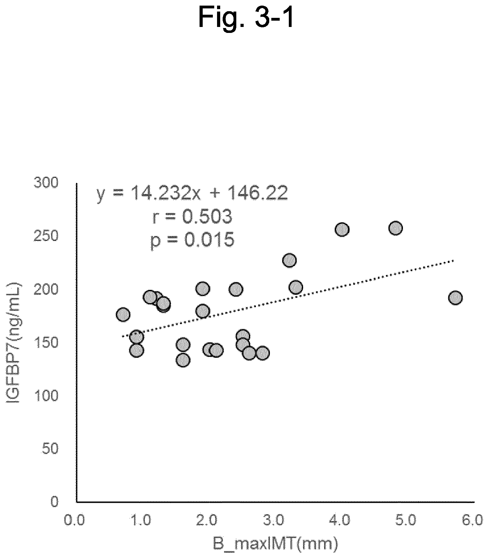

[Figure 3-1] Figure 3-1 is a graph showing the results of correlation analysis of the IGFBP7 value and the maxIMT value in the serum specimens of patients with arteriosclerosis-related disease (arteriosclerosis obliterans and myocardial infarction) in Example 3.

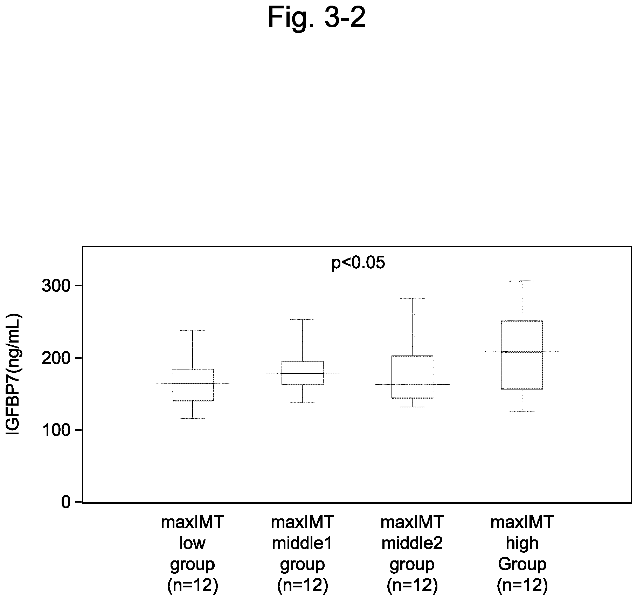

[Figure 3-2] Figure 3-2 is a graph showing the results of the trend analysis of the IGFBP7 value and the maxIMT value in the serum specimens of patients with aortic aneurysm (including thoracic aortic aneurysm, abdominal aortic aneurysm, and aortic aneurysm involving the complex onset in the chest and the abdomen) in Example 3.

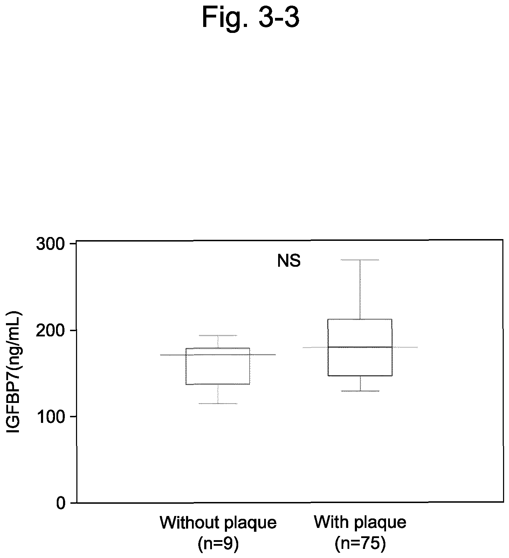

[Figure 3-3] Figure 3-3 is a graph showing the results of comparative analysis of the IGFBP7 value and the presence or absence of a carotid artery plaque in the serum specimens of patients with arteriosclerosis-related disease (aortic aneurysm, aortic dissection, arteriosclerosis obliterans, and myocardial infarction) in Example 3. "NS" indicates "not significant (no significant difference)."

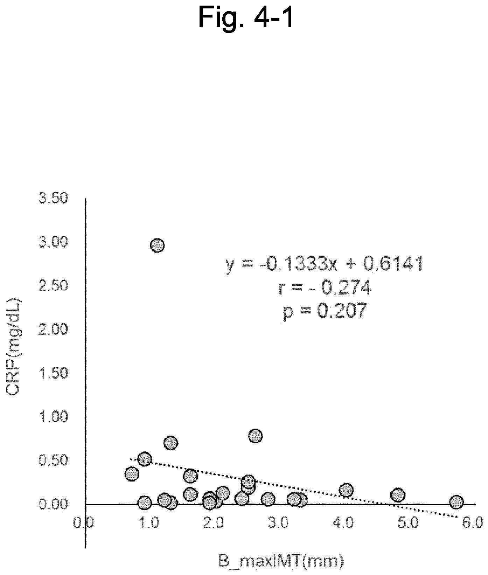

[Figure 4-1] Figure 4-1 is a graph showing the results of correlation analysis of the CRP value and the maxIMT value in the serum specimens of patients with arteriosclerosis-related disease (arteriosclerosis obliterans and myocardial infarction) in Comparative Example 1.

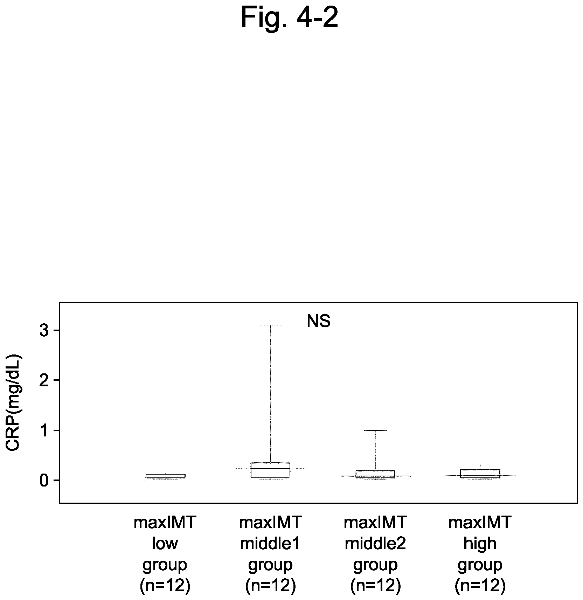

[Figure 4-2] Figure 4-2 is a graph showing the results of the trend analysis of the CRP value and the maxIMT value in the serum specimens of patients with aortic aneurysm (including thoracic aortic aneurysm, abdominal aortic aneurysm, and aortic aneurysm involving the complex onset in the chest and the abdomen) in Comparative Example 1. "NS" indicates "not significant (no significant difference)."

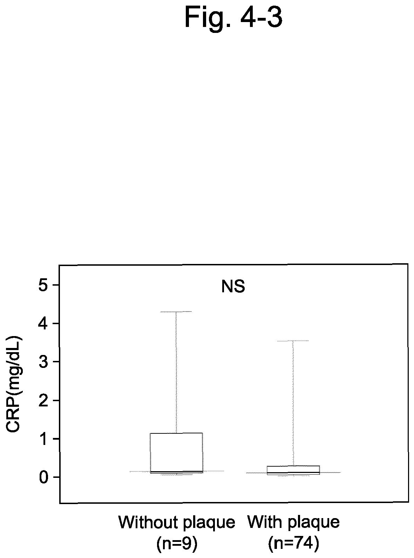

[Figure 4-3] Figure 4-3 is a graph showing the results of comparative analysis of the CRP value and the presence or absence of a carotid artery plaque in the serum specimens of patients with arteriosclerosis-related disease (aortic aneurysm, aortic dissection, arteriosclerosis obliterans, and myocardial infarction) in Comparative Example 1. "NS" indicates "not significant (no significant difference)."

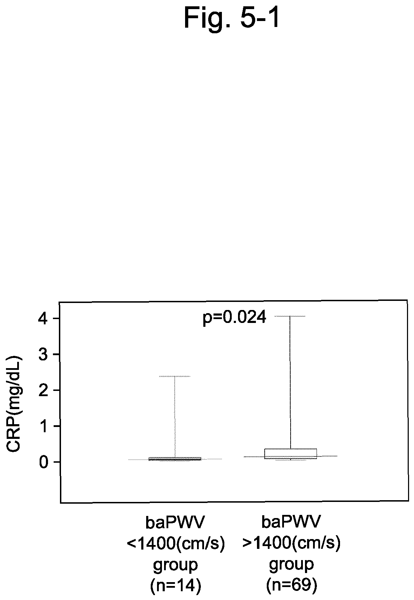

[Figure 5-1] Figure 5-1 is a graph showing the results of comparative analysis of the CRP value and the baPWV value (cutoff value: 1400 (cm/s)) in the serum specimens of patients with arteriosclerosis-related disease (aortic aneurysm, aortic dissection, arteriosclerosis obliterans, and myocardial infarction) in Comparative Example 2.

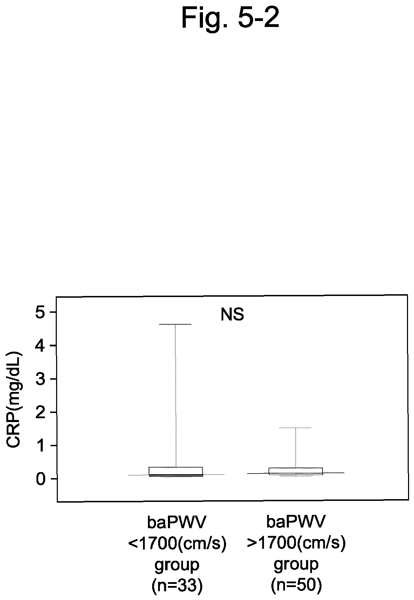

[Figure 5-2] Figure 5-2 is a graph showing the results of comparative analysis of the CRP value and the baPWV value (cutoff value: 1700 (cm/s)) in the serum specimens of patients with arteriosclerosis-related disease (aortic aneurysm, aortic dissection, arteriosclerosis obliterans, and myocardial infarction) in Comparative Example 2. "NS" indicates "not significant (no significant difference)."

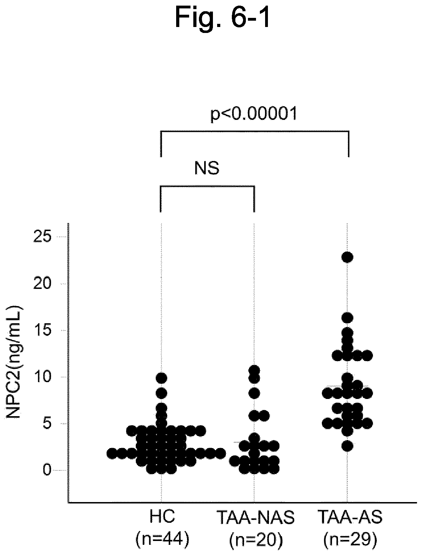

[Figure 6-1] Figure 6-1 is a graph showing the measurement results of the NPC2 value in serum specimens of healthy subjects, patients with non-atherosclerotic thoracic aortic aneurysm, and patients with atherosclerotic thoracic aortic aneurysm in Example 4. "HC" indicates healthy control (healthy subject), "TAA-NAS" indicates "Thoracic aortic aneurysm-non-atherosclerotic (non-atherosclerotic thoracic aortic aneurysm)," "TAA-AS" indicates "Thoracic aortic aneurysm-atherosclerotic (atherosclerotic thoracic aortic aneurysm)," and "NS" indicates "not significant (no significant difference)."

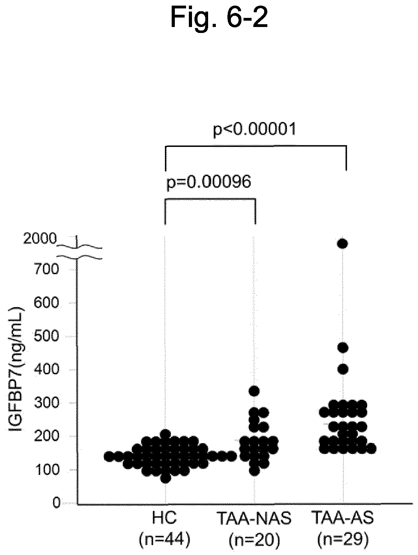

[Figure 6-2] Figure 6-2 is a graph showing the measurement results of the IGFBP7 value in serum specimens of healthy subjects, patients with non-atherosclerotic thoracic aortic aneurysm, and patients with atherosclerotic thoracic aortic aneurysm in Example 4. "HC" indicates healthy control (healthy subject), "TAA-NAS" indicates "Thoracic aortic aneurysm-non-atherosclerotic (non-atherosclerotic thoracic aortic aneurysm)," and "TAA-AS" indicates "Thoracic aortic aneurysm-atherosclerotic (atherosclerotic thoracic aortic aneurysm)."

### Description of Embodiments

For the purpose of identifying a marker that reflects the condition of arteriosclerosis, the present inventors have performed the following proteome analysis, using aortic media tissues of smooth muscle layers collected from 14 cases of aortic media of (non-vascular disease) control subjects and 29 cases of aortic media of atherosclerotic thoracic aortic aneurysm patients.

### - Tissue disruption and trypsin digestion

After collection of the tissues, the frozen tissues were pulverized using a bead pulverizer, and thereafter, Lys-C (Lysyl Endopeptidase) and trypsin were added to the resulting tissues. The obtained mixture was incubated at 37°C overnight for digestion. The obtained digested peptides were desalted using a C18 (octadecylsilyl) column to prepare an analysis sample.

### - Analysis

The analysis sample was separated using Nano LC (a nano-liquid chromatograph, Hitachi Nano Frontier, Hitachi High-Technologies Corporation), and was then subjected to tandem mass spectrometric (MS/MS) analysis using connected Triple TOF 5600 (SCIEX). From the measurement data, peptides/proteins were identified using Mascot database search (Matrix science), and were then quantified using 2DICAL (2 Dimensional Image Converted Analysis of Liquid chromatography mass spectrometry, MITSUI KNOWLEDGE INDUSTRY CO., LTD.) that is comparative quantification proteome analysis software, in which LC-mass spectrometry (MS) data were integrated and used. An atherosclerotic thoracic aortic aneurysm tissues were classified (depending on the stage of disease progression) into plural groups, and a comparison was then made among these plural classifications, so that proteins having quantitative differences between the plural classifications were searched. Moreover, a comparison was also made between proteome analysis data of a (non-vascular disease) control tissues and an atherosclerotic thoracic aortic aneurysm tissues, so that proteins having quantitative differences between the aortic media tissues of a (non-vascular disease) control group and the atherosclerotic thoracic aortic aneurysm group were searched. As a result, NPC2 and IGFBP7 were identified as proteins exhibiting significant increases between the classifications of the atherosclerotic thoracic aortic aneurysm groups and also, between the control group and the atherosclerotic thoracic aortic aneurysm group, thereby completing the present invention.

Besides, in the present invention, the markers of the present invention have been selected candidate factors not only by comparing proteome analysis data between the control group and the atherosclerotic thoracic aortic aneurysm group, but also by referring to differences in the proteome analysis data between non-atherosclerotic thoracic aortic aneurysm and atherosclerotic thoracic aortic aneurysm.

As described above, it has been confirmed that the expression of both NPC2 and IGFBP7 is significantly augmented in the aortic media tissues derived from patients with atherosclerotic thoracic aortic aneurysm caused by atherosclerosis, in comparison to in the aortic media tissues of control subjects. By using NPC2 or IGFBP7 as a single marker, arteriosclerosis-related disease and atherosclerosis can be detected. However, as shown in the Examples later, the two markers are more strongly related to each different indicator of arteriosclerosis (that is, as shown in Examples 1 and 2 later, NPC2 is a marker that is more strongly related to the presence or absence of a carotid artery plaque and the baPWV value, whereas, as shown in Example 3 later, IGFBP7 is a marker that is more strongly related to the maximum value of IMT of the carotid artery (hereinafter referred to as a "maxIMT value")). Accordingly, by combining NPC2 with IGFBP7, these markers provide an excellent detection sensitivity and more precise indicators reflecting the conditions of arteriosclerosis (stage of progression).

The present invention is characterized in that NPC2 and/or IGFBP7 are used as a marker for detecting arteriosclerosis-related disease or a marker for evaluating the stage of progression of arteriosclerosis.

In the present invention, the "marker" or "biomarker" means a molecule used as a measurement target of a sample obtained from a subject.

The marker found by the present invention comprises NPC2 and/or IGFBP7. Human NPC2 (i.e. Niemann-Pick disease type C2 protein) is a secretory protein having UniProt Accession No. P61916 (http://www.uniprot.org/uniprot/P61916). The amino acid sequence of human NPC2 is as set forth in SEQ ID NO: 1. In the amino acid sequence as set forth in SEQ ID NO: 1, the amino acid sequence portion consisting of the amino acids at positions 1 to 19 is a signal peptide, and the amino acid sequence portion consisting of the amino acids at positions 20 to 151 is a mature protein. Human NPC2 is a glycoprotein consisting of 132 amino acids, and is present abundantly in the epididymidis. Human NPC2 is also present in many other tissues. It is considered that NPC2 has cholesterol-binding ability, and that NPC2, together with NPC1, is associated with cholesterol excretion from lysosome. The loss-of-function of NPC2 causes Niemann-Pick disease type C involving abnormal accumulation of cholesterol in lysosome (Marie T. Vanier, Gilles Millat. Structure and function of the NPC2 protein. Biochimica et Biophysica Acta 2004; 1685: 14-21).

In the present invention, the NPC2 protein includes a protein consisting of an amino acid sequence having sequence identity of 70% or more, 75% or more, 80% or more, 85% or more, or 90% or more, more preferably 95% or more, 96% or more, 97% or more, 98% or more, or 99% or more, and most preferably 100%, to the amino acid sequence portion consisting of the amino acids at positions 20 to 151 in the amino acid sequence as set forth in SEQ ID NO: 1, and having cholesterol-binding activity and/or the activity of excreting cholesterol from lysosome, or not having the aforementioned activity, such as a loss-of-function mutant. The NPC2 protein further includes multimers (a dimer or more) of the aforementioned proteins.

On the other hand, human IGFBP7 (i.e. Insulin-like growth factor-binding protein 7) is a secretory protein having UniProt Accession No. Q16270 (http://www.uniprot.org/uniprot/Q16270). The amino acid sequence of human IGFBP7 is as set forth in SEQ ID NO: 2. In the amino acid sequence as set forth in SEQ ID NO: 2, the amino acid sequence portion consisting of the amino acids at positions 1 to 26 is a signal peptide, and the amino acid sequence portion consisting of the amino acids at positions 27 to 282 is a mature protein. IGFBP7 is expressed in many tissues such as heart, brain, placenta, lung, liver, skeletal muscle, kidney, pancreas, spleen, thymus, prostate, testis, ovary, small intestine, and colon. It has been known that, differing from other IGFBP proteins, the IGFBP7 protein has weak binding ability to an insulin-like growth factor (IGF) and strongly binds to insulin. It has been reported that IGFBP7 is associated with regulation of cell growth, apoptosis, cellular aging, and angiogenesis (Shuzhen Zhu, Fangying Xu, Jing Zhang, Wenjing Ruan, Maode Lai. Insulin-like growth factor binding protein-related protein 1 and cancer. Clinica Chimica Acta 2014; 431: 23-32).

In the present invention, the IGFBP7 protein includes a protein consisting of an amino acid sequence having sequence identity of 70% or more, 75% or more, 80% or more, 85% or more, or 90% or more, more preferably 95% or more, 96% or more, 97% or more, 98% or more, or 99% or more, and most preferably 100%, to the amino acid sequence portion consisting of the amino acids at positions 27 to 282 in the amino acid sequence as set forth in SEQ ID NO: 2, and having insulin-binding activity or not having the activity. The IGFBP7 protein further includes multimers (a dimer or more) of the aforementioned protein.

Besides, it is also possible to use the marker according to the present invention in combination with known markers reportedly associated with arteriosclerotic disease, including LDL cholesterol, HDL cholesterol, denatured LDL such as oxidized LDL, sdLDL (small dense LDL), C-reactive protein (CRP), Pentraxin3, and IL-6.

In the present invention, the "arteriosclerosis-related disease" means a disease developed due to arteriosclerosis. The disease that can be detected by the arteriosclerosis-related disease marker according to the present invention is not particularly limited, as long as it is a disease developed due to arteriosclerosis. Specific examples of such a disease may include myocardial infarction, peripheral arterial disease, aortic aneurysm, aortic dissection, stroke (atherothrombotic cerebral infarction, transient ischemic attack, etc.), renal artery stenosis, internal carotid artery stenosis, and angina pectoris. In the present description, arteriosclerosis and diseases related thereto are collectively referred to as "arteriosclerotic disease" in some cases. Moreover, various arteriosclerosis-related diseases, as mentioned above, may be said to be arteriosclerosis-related diseases that can be diagnosed, examined, evaluated or determined using the marker according to the present invention.

In the present invention, the method for detecting arteriosclerosis-related disease marker and the method of measuring an arteriosclerosis marker (i.e. a method of measuring a marker for evaluating the stage of progression of arteriosclerosis) comprise a step of measuring an NPC2 protein and/or an IGFBP7 protein. Upon the measurement of an NPC2 protein and/or an IGFBP7 protein in a sample, NPC2 and/or IGFBP7 serving as a glycoprotein(s) may be measured, or after the removal of a post-translational modification such as a sugar chain or phosphoric acid by a pre-treatment, the amino acid sequence portion(s) of NPC2 and/or IGFBP7 may be measured.

In the method for detecting arteriosclerosis-related disease, the marker according to the present invention is measured in a sample obtained from a subject, so that the arteriosclerosis-related disease of the subject can be detected. Accordingly, the method for detecting arteriosclerosis-related disease may be said to be a method of diagnosing, testing, evaluating or determining arteriosclerosis-related disease, or a method of collecting *in vitro* data for detecting arteriosclerosis-related disease, or the like.

As shown in Examples 1 to 3 later, the marker according to the present invention has been confirmed to have a strong relationship with carotid ultrasonography, baPWV examination, etc. as an indicator of arteriosclerosis. Thus, according to the method for evaluating the stage of progression of arteriosclerosis, namely, according to the method for evaluating the stage of the progression of arteriosclerosis, the same level of information as the stage of progression of arteriosclerosis in blood vessels obtained by carotid ultrasonography, baPWV examination, etc. can be grasped by only measuring the marker according to the present invention in a sample obtained from a subject. Therefore, the method for evaluating the stage of progression of arteriosclerosis may be said to be a method of diagnosing, testing, evaluating or determining arteriosclerosis, or a method of collecting *in vitro* data for detecting arteriosclerosis, or the like.

Examples of the animal that can be a test subject used in the method for detecting arteriosclerosis-related disease and the method for evaluating the stage of progression of arteriosclerosis may include mammals such as a human, a monkey, a bovine, a swine, a horse, a dog, a cat, sheep, a goat, a rabbit, a hamster, a Guinea pig, a mouse and a rat, and the animal used herein is preferably a human.

In addition, examples of the sample used in the method for detecting arteriosclerosis-related disease and the method for evaluating the stage of progression of arteriosclerosis may include serum, plasma, blood, urine, and saliva.

In the present invention, for example, the NPC2 protein level and/or the IGFBP7 protein level are measured *in vitro* in a sample collected from a patient suspected of suffering from arteriosclerosis, so that the possibility of the current progress of arteriosclerosis-related disease or arteriosclerosis, the risk (possibility) of the onset of arteriosclerosis-related disease in future, or the recurrence of arteriosclerosis-related disease can be determined, detected, or screened. When the NPC2 protein level and/or the IGFBP7 protein level in a patient specimen are compared with the level(s) in a healthy subject and as a result, when the NPC2 protein level and/or the IGFBP7 protein level in the patient specimen are higher than the level(s) in the healthy subject, it can be determined that it is highly likely that arteriosclerosis-related disease or arteriosclerosis currently progresses, that there is a risk (possibility) that arteriosclerosis-related disease will develop in future, or that arteriosclerosis-related disease has recurred.

Specifically, in accordance with the method for detecting arteriosclerosis-related disease and the method for evaluating the stage of progression of arteriosclerosis, the present invention further includes the following methods:
a method of detecting or screening the recurrence of arteriosclerosis-related disease by measuring the marker according to the present invention in a sample obtained from a subject having a risk of the recurrence of arteriosclerosis-related disease; and/or
a method of detecting or screening a subject having a risk of developing arteriosclerosis-related disease in future by measuring the marker according to the present invention in a sample obtained from the subject.

Moreover, the cutoff value of each marker for determining that it is highly likely that arteriosclerosis-related disease will develop or arteriosclerosis currently progresses is not limited, but examples of the cutoff value are the following. The following cutoff values are each calculated based on the ROC curve produced from the data shown in the Examples.

(i) Cutoff value of NPC2 based on Example 1 (comparative analysis with the presence or absence of a carotid artery plaque): 2.8 ng/mL (sensitivity: 68.0%; specificity: 22.2%); Cutoff value of NPC2 based on Example 2 (cutoff value of baPWV: 1400 cm/s): 2.3 ng/mL (sensitivity: 75.0%; specificity: 29.0%); and
   Cutoff value of NPC2 based on Example 2 (cutoff value of baPWV: 1700 cm/s): 3.4 ng/mL (sensitivity: 65.0%; specificity: 35.0%).
(ii) Cutoff value of IGFBP7 based on Example 3 (comparative analysis with the presence or absence of a carotid artery plaque): 178.0 ng/mL (sensitivity: 53.3%; specificity: 22.2%).

On the basis of these cutoff values, when the NPC2 protein level and/or the IGFBP7 protein level in a patient specimen are higher than the cutoff values, it can be determined that it is highly likely that arteriosclerosis-related disease will be developed, or that arteriosclerosis currently progresses.

The marker according to the present invention can also be used in the screening of a preventive or therapeutic agent for arteriosclerosis-related disease or arteriosclerosis, or the evaluation or determination of the effects of a preventive or therapeutic agent for arteriosclerosis-related disease or arteriosclerosis. For example, when the effects of a preventive or therapeutic agent for arteriosclerosis-related disease or arteriosclerosis are evaluated or determined, samples are collected from a test subject animal in need of prevention or treatment of arteriosclerosis-related disease or arteriosclerosis, before and after the corresponding preventive or therapeutic agent is administered to the test subject animal, or samples are collected from the test subject animal in two or more time points in chronological order, after administration of the preventive or therapeutic agent, and thereafter, a change over time in the NPC2 protein level and/or the IGFBP7 protein level in the samples is examined, so that the effects of the corresponding preventive or therapeutic agent can be evaluated or determined. When the formation or progression of an arteriosclerosis lesion is suppressed by administration of the corresponding preventive or therapeutic agent, an increase over time in the NPC2 protein level and/or the IGFBP7 protein level in the sample collected from the test subject animal tends to be suppressed, or the level(s) remain unchanged. On the other hand, when the formation or progression of the arteriosclerosis lesion is ameliorated by administration of the corresponding preventive or therapeutic agent, the NPC2 protein level and/or the IGFBP7 protein level in the sample collected from the test subject animal tends to be decreased over time.

As methods of measuring the marker protein according to the present invention, all of publicly known methods may be applied, as long as the methods are capable of specifically measuring an NPC2 protein and an IGFBP7 protein. Examples of the methods may include immunoassay and mass spectrometry. In a reagent used upon the measurement of the marker protein according to the present invention, an antibody, an aptamer, or the like can be used as a detection agent.

The immunoassay used herein is not particularly limited. Examples of the immunoassay may include various types of enzyme immunoassay, radioimmunoassay (RIA), enzyme-linked immunosorbent assay (ELISA), double monoclonal antibody sandwich assay, monoclonal-polyclonal antibody sandwich assay, an immunostaining method, an immunofluorescence method, a Western blotting method, a biotin-avidin method, an immunoprecipitation method, a gold colloidal aggregation method, an immunochromatography method, latex agglutination method (LA), and turbidimetric immunoassay (TIA).

In a reagent used in the immunoassay, an anti-NPC2 antibody and an anti-IGFBP7 antibody, which have already been commercially available, may be used as detection agents. Otherwise, such antibodies may be prepared according to a common method, on the basis of the amino acid sequence (SEQ ID NO: 1) of NPC2 and the amino acid sequence (SEQ ID NO: 2) of IGFBP7, both of which have been known. The antibody may be an antibody specifically recognizing the structure of the amino acid sequence of each of NPC2 and IGFBP7, or may also be an antibody specifically recognizing the entire structure including post-translational modification such as each sugar chain, disulfide bond, phosphorylation, etc.

The animal species or clone, from which the antibody is derived, is not particularly limited, as long as the antibody is capable of detecting the NPC2 protein or the IGFBP7 protein. Examples of the antibody may include antibodies derived from a rabbit, a goat, a mouse, a rat, a Guinea pig, a horse, sheep, a camel, a chicken and the like. The antibody may be either a monoclonal antibody or a polyclonal antibody. In addition, antibodies of all subclasses suitable for specific binding to the NPC2 protein and the IGFBP7 protein can be used. Recombinant antibodies or fragments such as a Fab, Fab' or F(ab')2 fragment can be naturally used.

As an agent for detecting arteriosclerosis-related disease or an agent for evaluating arteriosclerosis according to the present invention, an aptamer having binding ability to the NPC2 protein or the IGFBP7 protein can also be utilized. For the production of such an aptamer, a nucleic acid aptamer that is bindable DNA or RNA or a derivative thereof, or a peptide aptamer composed of amino acids, may be synthesized from the amino acid sequence (SEQ ID NO: 1) of NPC2 or the amino acid sequence (SEQ ID NO: 2) of IGFBP7, each of which has been known, according to a publicly known method, and may be then used. Upon the measurement, the binding of such an aptamer can be detected by a luminescence method, a fluorescence method, or a surface plasmon resonance method.

The mass spectrometry used herein is not particularly limited. A mass spectrometer, in which the ion source of electrospray ionization (ESI), matrix assisted laser desorption ionization (MALDI), surface enhanced laser desorption ionization (SELDI), etc. is combined with time-of-flight mass spectrometer (TOF), ion trap mass spectrometer (IT), Fourier-transform mass spectrometer (FT), etc., can be utilized. LC-MS, CE-MS, etc., in which a mass spectrometer is connected with a separation device such as high performance liquid chromatography (HPLC) or capillary electrophoresis (CE), can be used. Moreover, examples of a method of obtaining mass spectrometry data may include data-independent acquisition (DIA), data-dependent acquisition (DDA), and multiple reaction monitoring (MRM). The mass spectrometry also includes the case of performing stable isotope labeling on a sample, using iTRAQ reagent (SCIEX), etc.

Various types of reagents necessary for performing the detection of arteriosclerosis-related disease or evaluation of the stage of progression of arteriosclerosis of the present invention may have previously been packaged and prepared as a kit. For example, necessary reagents, such as (i) a monoclonal antibody or polyclonal antibody that is used as a capture antibody specific to the marker protein according to the present invention, (ii) an enzyme-labeled monoclonal antibody or polyclonal antibody that is used as a detection antibody specific to the marker protein according to the present invention, and (iii) a substrate solution, are provided as a kit.

### Examples

Hereinafter, the Examples of the present invention will be shown. However, the present invention is not limited to these Examples.

### [Example 1]

### Relationship of serum NPC2 value with maxIMT value and with presence and absence of plaque

Patients suffering from aortic aneurysm, aortic dissection, arteriosclerosis obliterans and myocardial infarction were used as subjects. The serum concentration of NPC2 was measured according to an ELISA method, and the obtained serum NPC2 value was compared with the maxIMT value of a carotid artery obtained by echo examination, and with plaque findings (the presence or absence of a plaque) obtained by carotid ultrasonography. The NPC2 value was measured using NPC2 ELISA Kit (Aviva Systems Biology) in accordance with protocols included with the kit. As a maxIMT value, the maximum wall thickness containing the plaque of the carotid artery was measured using Aplio Series manufactured by CANON MEDICAL SYSTEMS CORPORATION. A group in which the presence of a plaque was observed during the echo examination was defined to be a plaque group, whereas a group in which a clear plaque was not observed was defined to be a non-plaque group. These groups were used in determination of the presence or absence of a plaque.

More specifically, with regard to the measurement of the NPC2 concentration according to an ELISA method, a diluted serum specimen was added to a plate on which an anti-NPC2 antibody had been immobilized, and was then incubated. Thereafter, the specimen solution was removed, and a biotin-labeled anti-NPC2 antibody was then added, followed by incubation. After completion of the incubation, a washing operation was performed, and HRP (Horseradish peroxidase)-labeled avidin was then added, followed by incubation. Subsequently, a washing operation was performed, a TMB (3,3',5,5'-tetramethylbenzidine) solution was then added, and a coloration reaction was then carried out. Thereafter, a stop solution was added to the reaction mixture, and then, using a microplate reader (SpectraMax M2e, Molecular Devices), the measurement was carried out at a main wavelength of 450 nm and at a sub-wavelength of 540 nm. The concentration was calculated from the obtained data, based on a calibration curve produced from the measurement data of simultaneously measured each standard solution, and the concentration of NPC2 was then measured.

Correlation analysis of the serum NPC2 value and the maxIMT value was carried out on 23 cases of arteriosclerosis-related disease (14 cases of arteriosclerosis obliterans and 9 cases of myocardial infarction) used as subjects. As a result, the correlation coefficient of the serum NPC2 value and the maxIMT value was 0.151 (p = 0.491). (Figure 1-1: the B_maxIMT on the horizontal axis indicates maximum IMT at the bifurcation of carotid artery.)

Trend analysis of the serum NPC2 value and the maxIMT value was carried out on 48 cases of aortic aneurysm (including thoracic aortic aneurysm, abdominal aortic aneurysm, and aortic aneurysm involving the complex onset in the chest and the abdomen; the same applies in all of the Examples below). The 48 cases were divided into 4 groups, namely, 12 cases of low group, 12 cases of middle group 1, 12 cases of middle group 2, and 12 cases of high group, successively from the low maxIMT value side, and the serum NPC2 value in each group was then examined. Using a Jonckheere-Terpstra test, a significance test was carried out regarding tendency. As shown in Figure 1-2, the serum NPC2 value tends to increase, as the maxIMT value increases.

Comparative analysis of the serum NPC2 value and the presence or absence of a carotid artery plaque was carried out on a total of 84 cases (52 cases of aortic aneurysm, 10 cases of aortic dissection, 14 cases of arteriosclerosis obliterans, and 8 cases of myocardial infarction). The 84 cases were divided into two groups, namely, into a non-plaque group and a plaque group, and the serum NPC2 value in each group was then examined. For statistical processing, a Mann-Whitney U test was used. As shown in Figure 1-3, the serum NPC2 value was significantly high in the group having a carotid artery plaque (p = 0.013).

### [Example 2]

### Relationship of serum NPC2 value with baPWV value

Patients suffering from aortic aneurysm, aortic dissection, arteriosclerosis obliterans and myocardial infarction were used as subjects. The serum concentration of NPC2 was measured according to an ELISA method, and the obtained serum NPC2 value was compared with the baPWV value. The serum concentration of NPC2 was measured in accordance with the method described in Example 1. The baPWV value was measured using BP-203RPEII (Exceed) manufactured by Japan Colin Co., Ltd.

Comparative analysis of the serum NPC2 value and the baPWV value was carried out on a total of 85 cases (49 cases of aortic aneurysm, 11 cases of aortic dissection, 8 cases of arteriosclerosis obliterans, and 17 cases of myocardial infarction). The 85 cases were divided into two groups according to a cutoff value of 1400 (cm/s) or 1700 (cm/s) of baPWV, and the serum NPC2 value in each group was then examined. For statistical processing, a Mann-Whitney U test was used. As shown in Figures 2-1 and 2-2, the serum NPC2 value was significantly high in the group having a high baPWV value (in the case of a cutoff value of 1400 (cm/s), p = 0.014; and in the case of a cutoff value of 1700 (cm/s), p = 0.031).

Since the serum NPC2 value can significantly increase in the presence of a carotid artery plaque and in the high baPWV value group (Examples 1 and 2), it was suggested that the serum NPC2 value is likely to become a marker of the progression of arteriosclerosis and a marker of arteriosclerosis-related disease.

### [Example 3]

### Relationship of serum IGFBP7 value with maxIMT value and with presence and absence of plaque

The same patients as those in Example 1 were used as subjects. The serum concentration of IGFBP7 was measured according to an ELISA method, and the obtained serum IGFBP7 value was compared with the maxIMT value of a carotid artery obtained by echo examination, and with plaque findings (the presence or absence of a plaque) obtained by carotid ultrasonography. The IGFBP7 value was measured using ELISA Kit for Insulin Like Growth Factor Binding Protein 7 (Cloud-Clone) in accordance with protocols included with the kit. The measurement of the maxIMT value and determination of the presence or absence of a plaque were carried out in accordance with the methods described in Example 1.

Specifically, with regard to the measurement of the IGFBP7 concentration according to an ELISA method, a diluted serum specimen was added to a plate on which an anti-IGFBP7 antibody had been immobilized, and was then incubated. Thereafter, the specimen solution was removed, and a detection reagent A solution was then added, followed by incubation. After completion of the incubation, a washing operation was performed, and a detection reagent B solution was then added, followed by incubation. Subsequently, a washing operation was performed, a TMB solution was then added, and a coloration reaction was then carried out. Thereafter, a stop solution was added to the reaction mixture, and then, using a microplate reader (SpectraMax M2e, Molecular Devices), the measurement was carried out at a main wavelength of 450 nm and at a sub-wavelength of 540 nm. The concentration was calculated from the obtained data, based on a calibration curve produced from the measurement data of simultaneously measured each standard solution, and the concentration of IGFBP7 was then measured.

As a result of the correlation analysis of the serum IGFBP7 value and the maxIMT value, it was found that the serum IGFBP7 value was significantly correlated to the maxIMT value (p = 0.015), having a correlation coefficient of R = 0.503 (Figure 3-1: the B_maxIMT on the horizontal axis indicates maximum IMT at the bifurcation of carotid artery). In addition, the results of the trend analysis of the serum IGFBP7 value and the maxIMT value are as shown in Figure 3-2, and the serum IGFBP7 value tended to significantly increase, as the maxIMT value increased (p < 0.05). Moreover, the results of the comparative analysis of the serum IGFBP7 value and the presence or absence of a carotid artery plaque are as shown in Figure 3-3, and the serum IGFBP7 tended to increase in the group having a carotid artery plaque.

As mentioned above, the serum IGFBP7 value is significantly correlated to the maxIMT value of the carotid artery, and as the maxIMT value of the carotid artery increases, the serum IGFBP7 value becomes significantly high. Accordingly, it was suggested that the serum IGFBP7 value is likely to become a marker of the progression of arteriosclerosis and a marker of arteriosclerosis-related disease. Moreover, from the data of Examples 1 to 3, it was demonstrated that NPC2 and IGFBP7 have a stronger correlation with each different indicator of the progression of arteriosclerosis. That is to say, by combining these NPC2 and IGFBP7 with each other and then using them in a test, it is anticipated that it is possible to obtain more detailed information of the stage of progression of arteriosclerosis.

### [Comparative Example 1]

### Relationship of serum CRP value with maxIMT value and with presence or absence of a plaque

The same patients as those in Example 1 were used as subjects. The serum concentration of CRP was measured according to an ELISA method, and the obtained serum CRP value was compared with the maxIMT value of a carotid artery obtained by echo examination, and with plaque findings (the presence or absence of a plaque) obtained by carotid ultrasonography. As a CRP measuring reagent, C-reactive protein kit CRP-LATEX X2 "SEIKEN" NX (Denka Seiken Co., Ltd.) was used, and as measurement apparatuses, LABOSPECT 008 Hitachi Automatic Analyzer and 7180 Hitachi Automatic Analyzer (Hitachi High-Technologies Corporation) were used. The measurement of the maxIMT value and determination of the presence or absence of a plaque were carried out in accordance with the methods described in Example 1.

As a result of the correlation analysis of the serum CRP value and the maxIMT value, it was found that the correlation coefficient of the serum CRP value and the maxIMT value was -0.274 (p = 0.207) (Figure 4-1: the B_maxIMT on the horizontal axis indicates maximum IMT at the bifurcation of carotid artery). In addition, the results of the trend analysis of the serum CRP value and the maxIMT value are as shown in Figure 4-2, and no tendency was found between the serum CRP value and the maxIMT value. Moreover, the results of the comparative analysis of the serum CRP value and the presence or absence of a carotid artery plaque are as shown in Figure 4-3, and no relationship was found between the serum CRP value and the presence or absence of a carotid artery plaque.

### [Comparative Example 2]

### Relationship of serum CRP value with baPWV value

The same patients as those in Example 2 were used as subjects. The serum concentration of CRP was measured according to an ELISA method, and the obtained serum CRP value was compared with the baPWV value. The measurement of the serum concentration of CRP was carried out in accordance with the method described in Comparative Example 1, whereas the measurement of the baPWV value was carried out in accordance with the method described in Example 2.

As shown in Figures 5-1 and 5-2, in the case of a cutoff value of 1400 (cm/s), the serum CRP value was significantly high in the high baPWV value group (p = 0.024). However, in the case of a cutoff value of 1700 (cm/s), no relationship was found between the serum CRP value and the baPWV value.

From the aforementioned results of Examples 1 to 3 and Comparative Examples 1 and 2, it was demonstrated that both NPC2 and IGFBP7 are markers of the progression of arteriosclerosis and markers of arteriosclerosis-related disease, which are more useful than

### CRP.

### [Example 4]

### Comparison between non-atherosclerotic thoracic aortic aneurysm patients and atherosclerotic thoracic aortic aneurysm patients in terms of NPC2 and IGFBP7 in serum specimens (ELISA method)

In order to confirm the usefulness of NPC2 and IGFBP7 as arteriosclerosis-related disease markers, the NPC2 value and the IGFBP7 value were measured in the serums of non-atherosclerotic thoracic aortic aneurysm (hereditary connective tissue disease) patients and atherosclerotic thoracic aortic aneurysm patients. NPC2 was measured in accordance with the method described in Example 1, whereas IGFBP7 was measured in accordance with the method described in Example 3. For statistical processing, StatFlex ver 6.0 (Artec Co., Ltd.) was used, and as a significance test, a Mann-Whitney U test was used.

As shown in Figure 6-1, the NPC2 values in healthy subjects (44 cases), non-atherosclerotic thoracic aortic aneurysm patients (20 cases) and atherosclerotic thoracic aortic aneurysm patients (29 cases) were 2.91±2.01 ng/mL, 3.02±3.21 ng/mL, and 9.03±4.37 ng/mL, respectively. Thus, the NPC2 value was higher in the atherosclerotic thoracic aortic aneurysm patients, than in the non-atherosclerotic thoracic aortic aneurysm patients. Therefore, it was demonstrated that NPC2 is a useful marker of arteriosclerosis-related disease.

As shown in Figure 6-2, the IGFBP7 values in healthy subjects (44 cases), non-atherosclerotic thoracic aortic aneurysm patients (20 cases) and atherosclerotic thoracic aortic aneurysm patients (29 cases) were 139.20±28.47 ng/mL, 187.25±58.97 ng/mL, and 291.96±316.83 ng/mL, respectively. Thus, the IGFBP7 value was higher in the atherosclerotic thoracic aortic aneurysm patients, than in the non-atherosclerotic thoracic aortic aneurysm patients. Therefore, it was demonstrated that also IGFBP7 is a useful marker of arteriosclerosis-related disease.

### Industrial Applicability

By using NPC2 and/or IGFBP7 that are the novel marker(s) of the arteriosclerosis-related disease and progression of arteriosclerosis according to the present invention, the progression of arteriosclerosis and the onset of arteriosclerosis-related disease can be detected with high sensitivity. Therefore, a measurement reagent and a reagent kit, in which the marker of arteriosclerosis-related disease or the marker of the progression of arteriosclerosis according to the present invention is used, can be used in the screening or early diagnosis of the onset of arteriosclerosis-related disease or arteriosclerosis, the monitoring of the stage of progression of arteriosclerosis, evaluation of a risk of the recurrence of arteriosclerosis-related disease, evaluation of a risk of the onset of the disease in future, the development and evaluation of a preventive or therapeutic agent therefor, and the like. Further, the present invention can be utilized in the industry for producing the measurement reagent and the reagent kit.

All publications, patents and patent applications cited in the present description are incorporated herein by reference in their entirety.

## Claims

1. A method for detecting arteriosclerosis-related disease, comprising a step of measuring the following 1) and/or 2) in a sample obtained from a subject:
1) an NPC2 (Niemann-Pick disease type C2) protein, and/or
2) an IGFBP7 (Insulin-like growth factor-binding protein 7) protein.

2. A method for detecting or screening the recurrence of arteriosclerosis-related disease, comprising a step of measuring the following 1) and/or 2) in a sample obtained from a subject having a risk of the recurrence of arteriosclerosis-related disease:
1) an NPC2 (Niemann-Pick disease type C2) protein, and/or
2) an IGFBP7 (Insulin-like growth factor-binding protein 7) protein.

3. A method for detecting or screening a subject having a risk of developing arteriosclerosis-related disease in future, comprising a step of measuring the following 1) and/or 2) in a sample obtained from the subject:
1) an NPC2 (Niemann-Pick disease type C2) protein, and/or
2) an IGFBP7 (Insulin-like growth factor-binding protein 7) protein.

4. A method for evaluating the effects of a preventive or therapeutic agent for arteriosclerosis-related disease or arteriosclerosis, wherein the method comprises:
a step of measuring the following 1) and/or 2) in a sample obtained from a subject in need of the prevention or treatment of arteriosclerosis-related disease or arteriosclerosis, before administration of a preventive or therapeutic agent for arteriosclerosis-related disease or arteriosclerosis; and
a step of measuring the following 1) and/or 2) in a sample obtained from the subject, after administration of the preventive or therapeutic agent for arteriosclerosis-related disease or arteriosclerosis, or
the method comprises a step of measuring the following 1) and/or 2) in samples obtained, at two or more time points in chronological order, from a subject in need of the prevention or treatment of arteriosclerosis-related disease or arteriosclerosis, after administration of a preventive or therapeutic agent for arteriosclerosis-related disease or arteriosclerosis:
1) an NPC2 (Niemann-Pick disease type C2) protein, and/or
2) an IGFBP7 (Insulin-like growth factor-binding protein 7) protein.

5. The method according to any one of claims 1 to 4, wherein the arteriosclerosis-related disease is selected from the group consisting of myocardial infarction, peripheral arterial disease, aortic aneurysm, aortic dissection, atherothrombotic cerebral infarction, transient ischemic attack, renal artery stenosis, internal carotid artery stenosis, and angina pectoris.

6. A method for evaluating the stage of progression of arteriosclerosis comprising a step of measuring the following 1) and/or 2) in a sample obtained from a subject:
1) an NPC2 (Niemann-Pick disease type C2) protein, and/or
2) an IGFBP7 (Insulin-like growth factor-binding protein 7) protein.

7. The method according to any one of claims 1 to 6, wherein the sample is selected from the group consisting of serum, plasma, blood, urine, and saliva.

8. The method according to any one of claims 1 to 7, wherein the subject is an animal in need of the prevention or treatment of arteriosclerosis-related disease or arteriosclerosis, and the effects of the preventive or therapeutic agent for arteriosclerosis-related disease or arteriosclerosis are evaluated or determined using the NPC2 protein and/or the IGFBP7 protein as an indicator.

9. The method according to any one of claims 1 to 8, wherein the subject is a human.

10. The method according to any one of claims 1 to 9, wherein the measurement method is immunoassay or mass spectrometry.

11. A marker for detecting arteriosclerosis-related disease, comprising the following 1) and/or 2):
1) an NPC2 protein, and/or
2) an IGFBP7 protein.

12. The marker according to claim 11, wherein the arteriosclerosis-related disease is selected from the group consisting of myocardial infarction, peripheral arterial disease, aortic aneurysm, aortic dissection, atherothrombotic cerebral infarction, transient ischemic attack, renal artery stenosis, internal carotid artery stenosis, and angina pectoris.

13. A marker for evaluating the stage of progression of arteriosclerosis, comprising the following 1) and/or 2):
1) an NPC2 protein, and/or
2) an IGFBP7 protein.

14. A measurement reagent for detecting arteriosclerosis-related disease or for evaluating the stage of progression of arteriosclerosis, wherein the measurement reagent comprises an antibody or an aptamer for measuring the marker according to any one of claims 11 to 13.

15. The measurement reagent according to claim 14, wherein the antibody is a monoclonal antibody and/or a polyclonal antibody.

16. A reagent kit for detecting arteriosclerosis-related disease or for evaluating the stage of progression of arteriosclerosis, wherein the reagent kit includes the measurement reagent according to claim 14 or 15.

17. The marker, measurement reagent, or reagent kit according to any one of claims 11 to 16, wherein the sample used for detecting arteriosclerosis-related disease or for evaluating the stage of progression of arteriosclerosis is selected from the group consisting of serum, plasma, blood, urine, and saliva.

18. Use of the marker according to any one of claims 11 to 13 for screening a preventive or therapeutic agent for arteriosclerosis-related disease or arteriosclerosis.

19. Use of the marker according to any one of claims 11 to 13 for detecting or screening *in vitro* the recurrence of arteriosclerosis-related disease.

20. Use of the marker according to any one of claims 11 to 13 for detecting or screening *in vitro* a subject having a risk of developing arteriosclerosis-related disease in future.


SEQUENCE LISTING

<110>  EIKEN KAGAKU KABUSHIKI KAISHA
       NATIONAL CEREBRAL AND CARDIOVASCULAR CENTER

<120>  A MARKER FOR ARTERIOSCLEROSIS AND ARTERIOSCLEROSIS-RELATED DISEASES

<130>  PH-7840-PCT

<160>  2

<170>  PatentIn version 3.5

<210>  1
<211>  151
<212>  PRT
<213>  Homo sapiens

<400>  1

<210>  2
<211>  282
<212>  PRT

<213>  Homo sapiens

<400>  2

Met Glu Arg Pro Ser Leu Arg Ala Leu Leu Leu Gly Ala Ala Gly Leu
1               5                   10                  15

Leu Leu Leu Leu Leu Pro Leu Ser Ser Ser Ser Ser Ser Asp Thr Cys
            20                  25                  30

Gly Pro Cys Glu Pro Ala Ser Cys Pro Pro Leu Pro Pro Leu Gly Cys
        35                  40                  45

Leu Leu Gly Glu Thr Arg Asp Ala Cys Gly Cys Cys Pro Met Cys Ala
    50                  55                  60

Arg Gly Glu Gly Glu Pro Cys Gly Gly Gly Gly Ala Gly Arg Gly Tyr
65                  70                  75                  80

Cys Ala Pro Gly Met Glu Cys Val Lys Ser Arg Lys Arg Arg Lys Gly
                85                  90                  95

Lys Ala Gly Ala Ala Ala Gly Gly Pro Gly Val Ser Gly Val Cys Val
            100                 105                 110

Cys Lys Ser Arg Tyr Pro Val Cys Gly Ser Asp Gly Thr Thr Tyr Pro
        115                 120                 125

Ser Gly Cys Gln Leu Arg Ala Ala Ser Gln Arg Ala Glu Ser Arg Gly
    130                 135                 140

Glu Lys Ala Ile Thr Gln Val Ser Lys Gly Thr Cys Glu Gln Gly Pro
145                 150                 155                 160

Ser Ile Val Thr Pro Pro Lys Asp Ile Trp Asn Val Thr Gly Ala Gln
                165                 170                 175

Val Tyr Leu Ser Cys Glu Val Ile Gly Ile Pro Thr Pro Val Leu Ile
            180                 185                 190

Trp Asn Lys Val Lys Arg Gly His Tyr Gly Val Gln Arg Thr Glu Leu
        195                 200                 205

Leu Pro Gly Asp Arg Asp Asn Leu Ala Ile Gln Thr Arg Gly Gly Pro
    210                 215                 220

Glu Lys His Glu Val Thr Gly Trp Val Leu Val Ser Pro Leu Ser Lys
225                 230                 235                 240

Glu Asp Ala Gly Glu Tyr Glu Cys His Ala Ser Asn Ser Gln Gly Gln
                245                 250                 255

Ala Ser Ala Ser Ala Lys Ile Thr Val Val Asp Ala Leu His Glu Ile
            260                 265                 270

Pro Val Lys Lys Gly Glu Gly Ala Glu Leu
        275                 280